# EUROPEAN PATENT SPECIFICATION

(11) **EP 2 398 459 B1**
(45) Date of publication and mention of the grant of the patent: **17.10.2018**
(21) Application number: 10736127.1
(22) Date of filing: 22.01.2010
(51) Int. Cl.: A61K 9/08, A61K 9/06, A61K 31/195, A61K 31/155, A61K 31/415, A61P 17/00, A61P 23/00

(54) **TRANSDERMAL DELIVERY OF DICLOFENAC, CARBAMAZEPINE AND BENZYDAMINE**
TRANSDERMALE FREISETZUNG VON DICLOFENAC, CARBAMAZEPIN UND BENZYDAMIN
ADMINISTRATION TRANSDERMIQUE DE DICLOFÉNAC, DE CARBAMAZÉPINE ET DE BENZYDAMINE

(30) Priority: 30.01.2009 US 206399 P
(43) Date of publication of application: 28.12.2011
(73) Proprietor: Kydes Pharmaceuticals, Llc, Baltimore, MD 21227 (US)
(72) Inventor: DORDUNOO, Stephen, K., Baltimore, MD 21227 (US)
(74) Representative: FRKelly
(86) International application number: PCT/US2010/000157
(87) International publication number: WO 2010/087947

(56) References cited:
- WO-A1-2007/066148
- US-A- 4 711 906
- US-A1- 2004 068 007
- US-B1- 6 262 121
- S. BABOOTA ET AL.: 'Formulation and Evaluation of Once-a-Day Transdermal Gels of Diclofenac Diethylamine' METHODS AND FINDING IN EXPERIMENTAL AND CLINICAL PHARMACOLOGY vol. 28, no. 2, 2006, pages 109 - 114, XP009145444
- A. KOGAN ET AL.: 'Microemulsions as transdermal drug delivery vehicles' ADVANCED IN COLLOIDAL AND INTERFACE SCIENCE vol. 123-126, 2006, pages 369 - 385, XP027908800

## Description

This application claims priority from U.S. Provisional Application No. 61/206,399, filed January 30, 2009.

### FIELD OF THE INVENTION

The present invention generally relates to the field of formulation of drugs in solutions for topical applications.

### BACKGROUND OF THE INVENTION

Diclofenac is a widely dispensed drug owing to its well-known analgesic, anti-inflammatory, anti-pyretic, anti-arthritic, anti-phlogistic and anti-rheumatic properties. Brogden et al., Diclofenac sodium: a review of its pharmacological properties and therapeutic use in rheumatic diseases and pain of various origins, Drugs 20(1): 24-48 (1980). It is a non-steroidal anti-inflammatory drug ("NSAID"). Chemically, diclofenac is a derivative of phenylacetic acid and contains a carboxylic acid group. As such, diclofenac is a weak acid (with a p*Ka* value of around 4) and is a relatively lipophilic molecule. The small, lipophilic molecule can access and diffuse rapidly in generally all tissues and systems, e.g. it can be distributed in blood, muscle, skin, interstitial tissue, and synovial fluids.

Diclofenac is typically taken orally, in the form of tablets. Although attempts to make drug-effective solutions comprising diclofenac have been made, these attempts produce unsatisfactory medications, because the diclofenac is poorly soluble in solution. To improve its solubility, diclofenac is used in its salt form. The diclofenac salts customarily used are sodium, potassium or other alkali and alkaline earth metals, and salts of organic nature, such as the salts of diethylammonium (DEA), N)-(2-hydroxyethyl) pyrrolidine (DHEP), basic amino acids, such as lysine, arginine and ornithine, or other pharmacologically acceptable organic bases which have the ability to render the resulting salt somewhat more soluble in aqueous solutions. O'Connor & Corrigan, Comparison of the physicochemical properties of the hydroxyethyl pyrrolidine diethylamine and sodium salt forms of diclofenac, Int'l. J. Pharma. 222:281-293 (2001); Chiarini et al., pH-solubility relationship and partition coefficients for some anti-inflammatory arylaliphatic acids, Arch. Pharm. (Weinheim) 317:268-273 (1984); Fini et al., Diclofenac/N-(2-hydroxyethyl) pyrrolidine: a new salt for an old drug, Drug Exp. Clin. Res. 19:81-88 (1993).

These salts are often dissolved in DMSO, whose safety as a medical solvent is suspect. Two further problems limit the current uses of liquid application of diclofenac sodium. For one, the solubility of diclofenac as a salt is still fairly low. Further limiting is the fact that diclofenac salts tend to easily precipitate out of solution made with co-solvents such as ethanol and propylene glycol. For example, on topical application of a diclofenac sodium solution containing 10% ethanol is likely to precipitate on the skin with the evaporation of ethanol. Nishihata, et al., Percutaneous absorption of diclofenac in rats and humans: aqueous gel formulation, Int. J. Pharm., 46:1-7 (1988). Even in aqueous solutions, gels or creams, diclofenac sodium may precipitate on the evaporation of water, especially if the drug is not soluble in the non-volatile components of the preparation. This may explain why permeation of diclofenac sodium decreases when its concentration in the preparation is increased from 1 to 3%. Fergany A. Mohammed, Topical Permeation Characteristics of Diclofenac Sodium from NaCMC Gels in Comparison with Conventional Gel Formulations, Drug Dev. Ind. Pharm., 27(10), 1083-1097 (2001).

Use of diclofenac in solid form triggers other drawbacks. The drug's distribution is systemic and therefore the whole organism is exposed to large doses of the drug, even for indications, e.g. arthritis, where topical applications are more sensible, as the topically applied drug could effectively reach the target organ or site, without entering the whole system in large quantities. Systemic delivery, however, is not always desirable. Diclofenac is known to, for example, create ulcerogenic effects of diclofenac. Other NSAIDs are known to be concentration dependent and topical application with allow delivery of the required dosage without delivery of system wide large dosage. *See* Roth & Shainhouse, Efficacy and Safety of a Topical Diclofenac Solution in the Treatment of Primary Osteoarthritis of the Knee: A Randomized, Double-Blind, Vehicle-Controlled Clinical Trial. Arch. Intern. Med., 164:2017-2023 (2004).

Neuropathic pain is a common problem in our society affecting nearly 1.5% of the U.S. population. Carbamazepine is one of five medications approved by the U.S. Food and Drug Administration (FDA) for the treatment of neuropathic pain. Dobecki D.A. et al., Update on pharmacotheraphy guidelines for the treatment of neuropathic pain, Curr. Pain Headache Rep. 10(3):185-90 (2006). The situation with diclofenac and its unsatisfactory use in a topical liquid form is paralleled by certain other pain-relieving drugs. For example, carbamazepine is difficult to place in solution in effective doses and prevent its precipitation out of solution. The concentration and solubility problem are known also for another NSAID drug, benzydamine.

Benzydamine is a locally-acting nonsteroidal anti-inflammatory drug with local anaesthetic and analgesic properties providing both rapid and extended pain relief as well as a significant anti-inflammatory treatment for the painful inflammatory conditions of the mouth and throat. Turnbull RS, Benzydamine Hydrochloride (Tantum) in the management of oral inflammatory conditions, Journal of the Canadian Dental Association, 61(2):127-34 (1995). Benzydamine base is insoluble in water. It is available as the hydrochloride salt in topical aqueous sprays or gargles.

Accordingly, it would be desirable to produce liquid solutions having diclofenac, carbamazepine and benzydamine, where these drugs are at effective dosages in solution and are kept stably in solutions, at room temperature, for sufficient periods of time to allow for a reasonable commercial shelf-life.

### SUMMARY OF THE INVENTION

In accordance to one aspect, the invention provides a solution comprising:
a first active ingredient that is about 1.5% to about 20% w/v diclofenac-provided in a free acid or base as applicable or in a simple ester form (but not as a salt);
   a second active ingredient that is about 0.05% w/v to 10% w/v-lidocaine an acid stabilizer selected from among 2% w/v acetic acid and 3% w/v lactic acid;
   a solvent selected from among ethanol, ethoxydiglycol, dimethyl isosorbide, isopropyl myristate, isopropyl palmitate, glycofurol (Tetraglycol), dipropylene glycol, butylene glycol, propylene glycol, eucalyptol and glycerin; and
   an emollient;
wherein the solution is oily, can be stored at a temperature between about 2' C and 35° C, and, at that temperature, the active ingredients remain in solution without precipitation/ crystallization and chemically un-degraded for a period of time up to about two years.

Preferably the at least one first active ingredient is diclofenac at a concentration of between about 5% to about 10% w/v provided as a simple ester diclofenac, a free acid diclofenac or a combination of simple ester diclofenac and free acid diclofenac. Preferably the ester group is a straight chain, essentially un-substituted organic molecule comprising up to about 10 carbon atoms.

The solvent may be at least one from among about 5% w/v ethanol, ethoxydiglycol at a concentration of between about 15% to about 50% w/v, tetraglycol at a concentration of between about 10% to about 40% w/v and isopropyl myristate at a concentration of between about 10% to about 60% w/v.

The solvent may be about 5% w/v ethanol.

The solvent may be about 35% w/v ethoxydiglycol.

The solvent may be between about 30% to about 50% w/v isopropyl myristate.

The solution can further comprise a preservative, from among Parabens (methyl, propyl, butyl, isobutyl), sorbic acid, and phenoxyethanol.

The solution can further comprise at least one further ingredient from among an antioxidant, a preservative, a scenting agent and a surfactant.

The further ingredient may be eucalyptol, tocopheryl acetate, butylated hydroxytoluene, BHA, propyl gallate, ascorbyl palmitate, sorbic acid, eucalyptus oil, cardamom oil, linseed oil, volatile oils, or isopropyl myristate.

### DETAILED DESCRIPTION OF THE INVENTION

The invention provides a solution comprising certain NSAID drugs at a sufficiently concentration in a liquid or gel form to provide good therapeutic (analgesic, anti-inflammatory, anti-pyretic, anti-arthritic, anti-phlogistic and anti-rheumatic) effects. The solution is both physically and chemically stable for periods of time of many months or longer, up to at least about 24 months at room temperature. Contrary to the tried approaches, the present invention took the approach of starting with a free acid or, depending of the chemical nature of the drug, a base. Alternatively, the drug is provided as a simple ester.

The NSAID is diclofenac. However, making the free acid or simple ester diclofenac solutions at a high concentration remained challenging and a careful selection of solvents was required. At least for the solution made out of an acid form of diclofenac and further comprising a further drug in a base form, such as lidocaine, a carefully selected acid stabilizer was needed. The formulation of the free acid or simple ester forms of diclofenac was critical to achieve commercially desirable levels of concentration and stability for diclofenac.

The principles (drug provided as a free acid or base or a simple ester) and the formulations disclosed herein and shown to work with diclofenac are critical also in the making of concentrated and stable solutions of other compounds such as carbamazepine, naproxen, ibuprofen and benzydamine.

Accordingly, the invention is the making of a solution comprising (w:v) at least about 1.5% and up to about 20% of diclofenac. Preferably, the solution comprises 5% to 10% diclofenac. The diclofenac of this solution is either a free acid, or at least one of various simple esters, or some combination of these diclofenac forms.

Diclofenac is a derivative of phenylacetic acid and contains a carboxylic acid group. The structure and the formal name assigned by IUPAC for diclofenac is 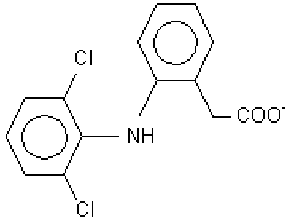 2-[(2,6-dichlorophenyl)amino]benzeneacetic acid.

The ester bond is on the carboxylic acid. A simple ester is any organic group of up to 10 carbons, preferably a straight chain organic group. The carbons of the ester group are preferably saturated, and, at least are essentially saturated, i.e., may contain a single instance of C=C (double bonded carbon). More preferably, the organic group is methyl, ethyl, or propyl.

These ester groups are attached to the diclofenac by any known method of esterification. For example, any of the following methods can be employed, each used as appropriate for particular starting components: trans-esterifications between other esters; Dieckmann condensation or Claisen condensation of esters carrying acidic α-protons; Favorskii rearrangement of α-haloketones in presence of base; nucleophilic displacement of alkyl halides with carboxylic acid salts; nucleophilic displacement of acyl halides with alcohols; Baeyer-Villiger oxidation of ketones with peroxides; Pinner reaction of nitriles with an alcohol.

It is critical that the formulation of diclofenac in solution includes an appropriate solvent. It must allow the drug to be of the desired, therapeutically effective concentration in solution, not precipitate out of solution for at least a month, preferably longer over a range of temperatures from about 2° C to about 35° C, and preserve the drug from chemical degradation. The solution must comprise at least about 5% diclofenac, preferably 10%. Solvents found satisfactory for this invention include: ethanol, ethoxydiglycol; dimethyl isosorbide; isopropyl myristate ("IPM"); isopropyl palmitate; tetrahydrofurfuryl alcohol (e.g. tetraglycol ("glycofurol")); dipropylene glycol; butylene glycol; Propylene glycol monocaprylate 90% (Capryol 90®) and glycerin. Any one solvent is present at a concentration raging from about 10% to about 50%. Preferably, the solvent is ethanol at about 5%; glycofurol at between from about 10% to about 50%, and more preferably at about between 10% to 25%; ethoxydiglycol at between from about 10% to about 50%, preferably at about between 15% to 30%; or a combination of glycofurol and Ethoxydiglycol, at a combined concentration of about between 45% to 65% where ethoxydiglycol, if present, is at least 20%; preferably 50%.

At least in cases when the diclofenac is provided as a free acid in combination with a base such as lidocaine, the solution contains a stabilizer of the diclofenac in its free acid form, i.e. a chemical that has a somewhat stronger affinity than diclofenac for any base in the solution. The stabilizer can be any organic acid of a molecular weight of up to about 400 daltons. Examples of preferred stabilizers include acetic acid and lactic acidAlthough not always necessary, the composition of the invention, comprising diclofenac, may comprise yet another active ingredient. It should be noted, that in the context of the invention, "active ingredient" is not something that is simply beneficial in some context, such as an emollient or water for hydration. In the context of the invention, an active ingredient is something generally recognized as a drug or medicament, having known effects on an organism, the effect being dose-dependent. It generally has a certain toxicity which is part of its mechanism of action. The "active ingredient" of the invention is preferably a drug having known activity as an NSAID, a neuropathic agent, an antifungal agent, a steroid, an antiseptic, local anesthetic, a vitamin, a vitamin analog, a topical antibiotic. Preferably, the active ingredients of the invention are an NSAID, diclofenac, and lidocaine.

The solution of the invention comprising diclofenac also comprises an emollient. ("An emollient" is somewhat synonymous with "a moisturizing agent," except that a moisturizing agent would also refer to a premade mixture having a moisturizing effect, while in the context of the invention, an emollient refers to individual compounds added to the mixture.) Typically, the emollient compound in the solution of the invention would be an oil, fatty acid or esters, such as, for example, at least one oil, a fatty acid or ester from among isopropyl myristate, isopropyl palmitate, sesame oil, shea butter, cocoa butter, linseed oil, Abyssinian oil, jojoba oil, palm oil, coconut oil, corn oil, cottonseed oil, cardamom oil, olive oil, rice bran oil or other vegetable oil(s). Preferred emollients include sesame oil, olive oil, and jojoba oil.

Accordingly, the solution of the invention has a somewhat oily, lubricating feel to it. The active ingredients are at a high concentration and stay stably in the solution for periods of time spanning at least about three months, six months, nine months, a year, fifteen months, twenty-one months, two years or more at temperatures ranging from about 2° C to about 35° C. Preferably, the solution stays stably in solution and un-degraded at about room temperature, i.e. between about 17° C and about 25° C.

An artisan skilled in the art will recognize that some of the compounds of the invention might be considered to have more than one role within the solution. For example, menthol is not only an active ingredient, but is also beneficial as a skin penetration enhancer; ethoxydiglycol, tetraglycol and IPM have major roles as solvents, but they also help penetrate the skin. The solution might further comprise various preservatives, antioxidants, surfactants and the like, including, for example, butylated hydroxyl toluene ("BTH"), vitamin E, parabens, ascorbic acid, sorbic acid, butylated hydroxyanisole (BHA), propyl gallate; phenoxyethanol, Phenonip® (Clariant UK, Ltd), benzyl alcohol, and/or about 2% lecithin.

In relation to the components in the solution presented in Example 1, below, the method of preparing the invention is as follows:

Thoroughly dissolve BHT and diclofenac and/or its ester in ethoxydiglycol. Nitrogen purge may be used during the dissolution and throughout the mixing with the other ingredients. In the following order, add lactic acid, lidocaine, menthol, anti-oxidants, sesame oil and the rest of the ingredients except isopropyl myristate (IPM). Stir until a clear solution is obtained. Then add IPM to volume. (Note that the isopropyl myristate is added in a volume sufficient to make the final volume to 100 ml ("sufficient quantity or QS"). When present, the isopropyl myristate is at least about 5% of the solution preferably 10% or more. The quantity of the ingredients in these examples, in grams or ml, are also an approximate indication of the percentage of that compound in the solution). Stir thoroughly and package the oil in a spray bottle or roll-on bottle. It will be recognized by an artisan skilled in the art, based on this description of the method, how to alter the order of addition of ingredients to allow for the absence of an ingredient or the use of an alternative or additional ingredients of the invention.

In the following Examples 1 through 7, preferred solutions are illustrated. However, the invention is not limited to only these examples. These solutions can also be prepared without inclusion of preservatives. The numbers refer to grams of the individual (for solids) and milliliters (for liquids). In addition to solubility at the desired levels of the active compounds (as illustrated in the examples), the stability of the active compounds in solution is tested by storage at room temperature (usually about 20° C), for periods of at least 1 year and periodic observation of the samples for precipitation. The compounds do not precipitate from solution.

### Example 1

| **Ingredient** | **Quantity (% w/v)** | **Reason for inclusion** | **Alternative ingredients** |
|---|---|---|---|
| Diclofenac acid | 5 | Active | Naproxen, ibuprofen, benzydamine, meloxicam and other NSAIDS |
| Lidocaine | 5 | Active | Prilocaine, bupivacaine and other local anesthetics |
| Menthol | 5 | Active/Penetration enhancer | Other mono-terpenes |
| Isopropyl myristate | QS | Penetration enhancer, emollient | Other esters of fatty acids |
| Ethoxydiglycol | 25 | Solvent | Other glycol ethers |
| Sesame oil | 10 | Emollient | Other vegetable oils |
| Lactic acid | 3 | Stabilizer | Acetic and propionic acids |
| Ethanol | 5 | Solvent | Other low molecular weight alcohols |
| Butylated hydroxyl-toluene | 0.05 | Anti-oxidant | BHA, propyl gallate |
| Tocopheryl acetate | 0.1 | Anti-oxidant | Ascorbyl palmitate, sorbic acid |
| Eucalyptol | 1.0 | Scenting agent | Eucalyptus oil, cardamom oil, linseed oil and other volatile oils |
| TOTAL | 100 | | |

### Reference Example 2

| **Ingredient** | **Quantity (%w/v)** | **Reason for inclusion** | **Alternative ingredients** |
|---|---|---|---|
| Diclofenac acid | 5 | Active | Naproxen, ibuprofen, benzydamine, meloxicam and other NSAIDS |
| Carbamazepine | 1.5 | Active | Prilocaine, bupivacaine and other local anesthetics |
| Menthol | 10 | Active/Penetration enhancer | Other mono-terpenes |
| Isopropyl myristate | QS | Penetration enhancer, emollient | Fatty acid esters |
| Tetraglycol | 30 | Solvent | Other glycols |
| Ethoxydiglycol | 25 | Solvent | Other glycol ethers |
| Sesame oil | 10 | Emollient | Other vegetable oils |
| Ethanol | 5 | Solvent | Other low molecular weight alcohols |
| Butylated hydroxyl-toluene | 0.05 | Anti-oxidant | BHA, propyl gallate |
| Tocopheryl acetate | 0.1 | Anti-oxidant | Ascorbyl palmitate, sorbic acid |
| Eucalyptol | 1.0 | Scenting agent | Eucalyptus oil, cardamom oil, linseed oil and other volatile oils |
| TOTAL | 100 | | |

### Reference Example 3

| **Ingredient** | **Quantity (%w/v)** | **Reason for inclusion** | **Alternative ingredients** |
|---|---|---|---|
| Diclofenac acid | 10.0 | Active | Naproxen, ibuprofen, benzydamine, meloxicam and other NSAIDS |
| Carbamazepine | 1.5 | Active | Prilocaine, bupivacaine and other local anesthetics |
| Menthol | 5.0 | Active/Penetration enhancer | Other mono-terpenes |
| Isopropyl myristate | QS | Penetration enhancer, emollient | Other esters of fatty acids |
| Tetraglycol | 25.0 | Solvent | Other glycols |
| Ethoxydiglycol | 25.0 | Solvent | Other glycol ethers |
| Sesame oil | 10.0 | Emollient | Other vegetable oils |
| Ethanol | 5.0 | Solvent | Other low molecular weight alcohols |
| Butylated hydroxyl-toluene | 0.05 | Anti-oxidant | BHA, propyl gallate |
| Tocopheryl acetate | 0.1 | Anti-oxidant | Ascorbyl palmitate, sorbic acid |
| Eucalyptol | 1.0 | Scenting agent | Eucalyptus oil, cardamom oil, linseed oil and other volatile oils |
| TOTAL | 100 | | |

### Reference Example 4

| **Ingredient** | **Quantity (%w/v)** | **Reason for inclusion** | **Alternative ingredients** |
|---|---|---|---|
| Diclofenac acid | 10.0 | Active | Naproxen, ibuprofen, benzydamine, meloxicam and other NSAIDS |
| Menthol | 10.0 | Active/Penetration enhancer | Other mono-terpenes |
| Isopropyl myristate | QS | Penetration enhancer, emollient | Other esters of fatty acids |
| Ethoxydiglycol | 25.0 | Solvent | Other glycol ethers |
| Sesame oil | 10.0 | Emollient | Other vegetable oils |
| Butylated hydroxyl-toluene | 0.05 | Anti-oxidant | BHA, propyl gallate |
| Tocopheryl acetate | 0.1 | Anti-oxidant | Ascorbyl palmitate, sorbic acid |
| Eucalyptol | 1.0 | Scenting agent | Eucalyptus oil, cardamom oil, linseed oil and other volatile oils |
| TOTAL | 100 | | |

### Example 5

| **Ingredient** | **Quantity (%w/v)** | **Reason for inclusion** | **Alternative ingredients** |
|---|---|---|---|
| Diclofenac acid | 5 | Active | Naproxen, ibuprofen, benzydamine, meloxicam and other NSAIDS |
| Lidocaine | 5 | Active | Prilocaine, bupivacaine and other local anesthetics |
| Menthol | 5 | Active/Penetration enhancer | Other mono-terpenes |
| Methyl salicylate | 15 | Active | Other salicylates |
| Isopropyl myristate | QS | Penetration enhancer, emollient | Other esters of fatty acids |
| Ethoxydiglycol | 25 | Solvent | Other glycol ethers |
| Sesame oil | 10 | Emollient | Other vegetable oils |
| Lactic acid | 3 | Stabilizer | Acetic and propionic acids |
| Butylated hydroxyl-toluene | 0.05 | Anti-oxidant | BHA, propyl gallate |
| Tocopheryl acetate | 0.1 | Anti-oxidant | Ascorbyl palmitate, sorbic acid |
| Eucalyptol | 1.0 | Scenting agent | Eucalyptus oil, cardamom oil, linseed oil and other volatile oils |
| TOTAL | 100 | | |

### Example 6

| **Ingredient** | **Quantity (%w/v)** | **Reason for inclusion** | **Alternative ingredients** |
|---|---|---|---|
| Diclofenac acid | 5 | Active | Naproxen, ibuprofen, benzydamine, meloxicam and other NSAIDS |
| Lidocaine | 5 | Active | Prilocaine, bupivacaine and other local anesthetics |
| Menthol | 5 | Active/Penetration enhancer | Other mono-terpenes |
| Isopropyl myristate | QS | Penetration enhancer, emollient | Other esters of fatty acids |
| Ethoxydiglycol | 25 | Solvent | Other glycol ethers |
| Sesame oil | 10 | Emollient | Other vegetable oils |
| Lactic acid | 3 | Stabilizer | Acetic and propionic acids |
| Butylated hydroxyl-toluene | 0.05 | Anti-oxidant | BHA, propyl gallate |
| Tocopheryl acetate | 0.1 | Anti-oxidant | Ascorbyl palmitate, sorbic acid |
| Eucalyptol | 5.0 | Solvent/scenting agent | Eucalyptus oil, cardamom oil, linseed oil and other volatile oils |
| TOTAL | 100 | | |

### Example 7

| **Ingredient** | **Quantity (%w/v)** | **Reason for inclusion** | **Alternative ingredients** |
|---|---|---|---|
| Diclofenac acid | 5 | Active | Naproxen, ibuprofen, benzydamine, meloxicam and other NSAIDS |
| Diclofenac ester | 5 | Active | Naproxen, Ibuprofen, benzydamine, meloxicam and other NSAIDS |
| Lidocaine | 5 | Active | Prilocaine, bupivacaine and other local anesthetics |
| Menthol | 5 | Active/Penetration enhancer | Other mono-terpenes |
| Isopropyl myristate | QS | Penetration enhancer, emollient | Other esters of fatty acids |
| Ethoxydiglycol | 25 | Solvent | Other glycol ethers |
| Sesame oil | 10 | Emollient | Other vegetable oils |
| Lactic acid | 3 | Stabilizer | Acetic and propionic acids |
| Butylated hydroxyl-toluene | 0.05 | Anti-oxidant | BHA, propyl gallate |
| Tocopheryl acetate | 0.1 | Anti-oxidant | Ascorbyl palmitate, sorbic acid |
| Eucalyptol | 5.0 | Solvent/scenting agent | Eucalyptus oil, cardamom oil, linseed oil and other volatile oils |
| TOTAL | 100 | | |

### Reference Example 8

| **Ingredient** | **Quantity (%w/v)** | **Reason for inclusion** | **Alternative ingredients** |
|---|---|---|---|
| Naproxen | 7.5 | Active | Diclofenac, ibuprofen, benzydamine, meloxicam and other NSAIDS |
| Carbamazepine | 1.5 | Active | Prilocaine, bupivacaine and other local anesthetics |
| Menthol | 10 | Active/Penetration enhancer | Other mono-terpenes |
| Isopropyl myristate | QS | Penetration enhancer, emollient | Fatty acid esters |
| Tetraglycol | 30 | Solvent | Other glycols |
| Ethoxydiglycol | 25 | Solvent | Other glycol ethers |
| Sesame oil | 10 | Emollient | Other vegetable oils |
| Ethanol | 5 | Solvent | Other low molecular weight alcohols |
| Butylated hydroxyl-toluene | 0.05 | Anti-oxidant | BHA, propyl gallate |
| Tocopheryl acetate | 0.1 | Anti-oxidant | Ascorbyl palmitate, sorbic acid |
| Eucalyptol | 1.0 | Scenting agent | Eucalyptus oil, cardamom oil, linseed oil and other volatile oils |
| TOTAL | 100 | | |

### Reference Example 9

| **Ingredient** | **Quantity (%w/v)** | **Reason for inclusion** | **Alternative ingredients** |
|---|---|---|---|
| Ibuprofen | 15 | Active | Naproxen, diclofenac, benzydamine, meloxicam and other NSAIDS |
| Carbamazepine | 1.5 | Active | Prilocaine, bupivacaine and other local anesthetics |
| Menthol | 10 | Active/Penetration enhancer | Other mono-terpenes |
| Isopropyl myristate | QS | Penetration enhancer, emollient | Fatty acid esters |
| Tetraglycol | 30 | Solvent | Other glycols |
| Ethoxydiglycol | 25 | Solvent | Other glycol ethers |
| Sesame oil | 10 | Emollient | Other vegetable oils |
| Ethanol | 5 | Solvent | Other low molecular weight alcohols |
| Butylated hydroxyl-toluene | 0.05 | Anti-oxidant | BHA, propyl gallate |
| Tocopheryl acetate | 0.1 | Anti-oxidant | Ascorbyl palmitate, sorbic acid |
| Eucalyptol | 1.0 | Scenting agent | Eucalyptus oil, cardamom oil, linseed oil and other volatile oils |
| TOTAL | 100 | | |

### Example 10

The penetration and absorption of the drug(s) from solution is determined by measuring the amount of active agent remaining on a skin after a determined amount of the solution is applied and a fixed period of time elapsed. The applied solution is removed by rubbing an alcohol-wetted cotton swab or equivalent on the treated skin, extracting organic material from the cotton swab, and the quantity of the active ingredient swabbed is measured by measuring its peak area, as revealed after HPLC analysis. Typically the elapsed time between application and swabbing the skin is 0.25 to 10 hours (1 to 6 hours preferred). A skilled artisan recognizes that standard controls typical in the scientific method apply. For example, the HPLC peaks are compared not only after the elapsed time vs. applied material, but also as a percentage of material recovered of a cotton swab immediately after the solution was applied to the skin. Statistically significant repeats are included in the analysis and the analysis is performed on the skin of a number of individuals.

### Example 11

Solutions containing diclofenac acid and lidocaine as mentioned in the Examples above, if prepared without inclusion of acid stabilizer, may possibly precipitate or re-crystallize on storage, possibly due to the formation of diclofenac lidocaine salt. Addition of acids such as acetic or lactic at 1:1 molar equivalents to lidocaine or higher is necessary for prevention of precipitation of diclofenac from solution.

| Composition | Acid stabilizer and amount | Precipitation |
|---|---|---|
| Example 1 | Without lactic acid | +++ |
| Example 1 | Acetic acid 1% | + |
| Example 1 | Acetic acid 2% | - (no precipitation) |
| Example 1 | Lactic acid 1% | ++ |
| Example 1 | Lactic acid 2% | + |
| Example 1 | Lactic acid 3% | - (no precipitation) |

The concentrations of chemicals are often herein described as percentages (%). Unless specifically indicated otherwise or clearly otherwise in the context, the percentages are of liquids and therefore the percentage is of a volume: "/v." What is added to the solution is either a liquid or a quantity of a dry material, so, as the context requires the units are "w/v" or "v/v." In case of uncertainty, the percentages are w/v.

The use of the terms "a" and "an" and "the" and similar referents in the context of describing the invention (especially in the context of the following claims) are to be construed to cover both the singular and the plural, unless otherwise indicated herein or clearly contradicted by context. Recitation of ranges of values herein are merely intended to serve as a shorthand method of referring individually to each separate value falling within the range, unless otherwise indicated herein, and each separate value is incorporated into the specification as if it were individually recited herein. The word "about," when accompanying a numerical value, is to be construed as indicating a deviation of up to and inclusive of 10% from the stated numerical value. All methods described herein can be performed in any suitable order unless otherwise indicated herein or otherwise clearly contradicted by context. The use of any and all examples, or exemplary language (e. g., "such as") provided herein, is intended merely to better illuminate the invention and does not pose a limitation on the scope of the invention unless otherwise claimed. No language in the specification should be construed as indicating any non-claimed element as essential to the practice of the invention.

The use of Trade names, for any chemical compound and the names of a supply company are used herein only as examples of the compound - - the invention is not limited to the compound from any particular source.

## Claims

1. A solution comprising:
a first active ingredient that is about 1.5% to about 20% w/v diclofenac provided in a free acid or base as applicable or in a simple ester form (but not as a salt);
a second active ingredient that is about 0.05% w/v to 10% w/v lidocaine
an acid stabilizer selected from among 2% w/v acetic acid and 3% w/v lactic acid;
a solvent selected from among ethanol, ethoxydiglycol, dimethyl isosorbide, isopropyl myristate, isopropyl palmitate, glycofurol (Tetraglycol), dipropylene glycol, butylene glycol, propylene glycol, eucalyptol and glycerin; and
an emollient;
wherein the solution is oily, can be stored at a temperature between about 2' C and 35° C, and, at that temperature, the active ingredients remain in solution without precipitation/ crystallization and chemically un-degraded for a period of time up to about two years.

2. The solution of claim 1, wherein the at least one first active ingredient is diclofenac at a concentration of between about 5% to about 10% w/v provided as a simple ester diclofenac, a free acid diclofenac or a combination of simple ester diclofenac and free acid diclofenac.

3. The solution of claim 2, wherein the ester group is a straight chain, essentially un-substituted organic molecule comprising up to about 10 carbon atoms.

4. The solution of claim 5 1, wherein the solvent is at least one from among about 5% w/v ethanol, ethoxydiglycol at a concentration of between about 15% to about 50% w/v, tetraglycol at a concentration of between about 10% to about 40% w/v and isopropyl myristate at a concentration of between about 10% to about 60% w/v.

5. The solution of claim 4, wherein the solvent is about 5% w/v ethanol.

6. The solution of claim 4, wherein the solvent is about 35% w/v ethoxydiglycol.

7. The solution of claim 4, wherein the solvent is between about 30% to about 50% w/v isopropyl myristate.

8. The solution of claim 1, further comprising a preservative, from among Parabens (methyl, propyl, butyl, isobutyl), sorbic acid, and phenoxyethanol.

9. The solution of claim 1, further comprising at least one further ingredient from among an antioxidant, a preservative, a scenting agent and a surfactant.

10. The solution of claim 9, where the further ingredient is eucalyptol, tocopheryl acetate, butylated hydroxytoluene, BHA, propyl gallate, ascorbyl palmitate, sorbic acid, eucalyptus oil, cardamom oil, linseed oil, volatile oils, or isopropyl myristate.

## Patentansprüche

1. Lösung, umfassend:
einen ersten Wirkstoff, der aus etwa 1,5 % bis etwa 20 % Gew./Vol. Diclofenac besteht, bereitgestellt als freie Säure oder Base, wie anwendbar, oder in einer einfachen Esterform (jedoch nicht als Salz);
einen zweiten Wirkstoff, der aus etwa 0,05 % Gew./Vol. bis 10 % Gew./Vol. Lidocain besteht
ein Säurestabilisator, ausgewählt aus 2 % Gew./Vol. Essigsäure und 3 % Gew./Vol. Milchsäure;
ein Lösungsmittel, ausgewählt aus Ethanol, Ethoxydiglykol, Dimethylisosorbid, Isopropylmyristat, Isopropylpalmitat, Glycofurol (Tetraglycol), Dipropylenglykol, Butylenglykol, Propylenglykol, Eucalyptol und Glycerin; und
einen Weichmacher;
wobei es sich um ein ölige Lösung handelt, die bei einer Temperatur zwischen etwa 2 °C und 35 °C gelagert werden kann, und wobei bei dieser Temperatur die Wirkstoffe für einen Zeitraum von ungefähr zwei Jahren ohne Ausfällung/Kristallisation in Lösung bleiben und nicht chemisch abgebaut werden.

2. Lösung nach Anspruch 1, wobei der mindestens eine erste Wirkstoff Diclofenac bei einer Konzentration von zwischen etwa 5 % bis etwa 10 % Gew./Vol. ist, bereitgestellt als ein einfacher Ester von Diclofenac, eine freie Säure von Diclofenac oder eine Kombination von einfachem Ester von Diclofenac und freier Säure von Diclofenac.

3. Lösung nach Anspruch 2, wobei die Estergruppe ein geradkettiges, im Wesentlichen nicht-substituiertes organisches Molekül ist, das bis zu etwa 10 Kohlenstoffatome umfasst.

4. Lösung nach Anspruch 1, wobei das Lösungsmittel mindestens eines von etwa 5 % Gew./Vol. Ethanol, Ethoxydiglykol in einer Konzentration von etwa 15 % bis etwa 50% Gew./Vol., Tetraglykol in einer Konzentration von etwa 10 % bis etwa 40 % Gew./Vol. und Isopropylmyristat in einer Konzentration von etwa 10 % bis etwa 60 % Gew./Vol ist.

5. Lösung nach Anspruch 4, wobei das Lösungsmittel etwa 5 % Gew./Vol. Ethanol ist.

6. Lösung nach Anspruch 4, wobei das Lösungsmittel etwa 35 % (Gew./Vol.) Ethoxydiglykol ist.

7. Lösung nach Anspruch 4, wobei das Lösungsmittel von etwa 30 % bis etwa 50 % Gew./Vol. Isopropylmyristat ist.

8. Lösung nach Anspruch 1, ferner umfassend ein Konservierungsmittel von Parabenen (Methyl-, Propyl-, Butyl-, Isobutyl-), Sorbinsäure und Phenoxyethanol.

9. Lösung nach Anspruch 1, ferner umfassend mindestens einen weiteren Inhaltsstoff von einem Antioxidationsmittel, einem Konservierungsmittel, einem Duftmittel und einem Tensid.

10. Lösung nach Anspruch 9, wobei der weitere Inhaltsstoff Eukalyptol, Tocopherylacetat, butyliertes Hydroxytoluol, BHA, Propylgallat, Ascorbylpalmitat, Sorbinsäure, Eukalyptusöl, Kardamomöl, Leinsamenöl, ätherische Öle oder Isopropylmyristat ist.

## Revendications

1. Solution comprenant :
un premier principe actif qui est d'environ 1,5 à environ 20 % p/v de diclofénac sous forme d'acide libre ou de base comme applicable ou d'ester simple (mais pas sous forme de sel) ;
un second principe actif qui est d'environ 0,05 à 10 % p/v de lidocaïne
un stabilisant acide choisi parmi 2 % p/v d'acide acétique et 3 % p/v d'acide lactique ;
un solvant choisi parmi l'éthanol, l'éthoxydiglycol, le diméthylisorbide, le myristate d'isopropyle, le palmitate d'isopropyle, le glycofurol (Tétraglycol), le dipropylène glycol, le butylène glycol, le propylène glycol, l'eucalyptol et le glycérol ; et
un émollient ;
où la solution est huileuse, peut être stockée à une température entre environ 2 et 35°C, et, à cette température, les principes actifs restent en solution sans précipitation/cristallisation et chimiquement non dégradés pendant une durée jusqu'à environ deux ans.

2. Solution selon la revendication 1, dans laquelle ledit au moins premier principe actif est le diclofénac à une concentration d'environ 5 à environ 10 % p/v sous forme de diclofénal ester simple, diclofénac acide libre ou une combinaison de diclofénal ester simple et acide libre.

3. Solution selon la revendication 2, dans laquelle le groupe ester est une molécule organique à chaîne droite, sensiblement non substituée comprenant jusqu'à environ 10 atomes de carbone.

4. Solution selon la revendication 1, dans laquelle le solvant est au moins choisi parmi environ 5 % p/v d'éthanol, l'éthoxydigycol à une concentration d'environ 15 à environ 50 % p/v, le tétraglycol à une concentration d'environ 10 à environ 40 % p/v et le myristate d'isopropyle à une concentration d'environ 10 à environ 60 % p/v.

5. Solution selon la revendication 4, dans laquelle le solvant est environ 5 % p/v d'éthanol.

6. Solution selon la revendication 4, dans laquelle le solvant est environ 35 % p/v d'éthoxydiglycol.

7. Solution selon la revendication 4, dans laquelle le solvant est d'environ 30 à environ 50 % p/v de myristate d'isopropyle.

8. Solution selon la revendication 1, comprenant en outre un conservateur, parmi les parabènes (de méthyle, propyle, butyle, isobutyle), l'acide sorbique et le phénoxyéthanol.

9. Solution selon la revendication 1, comprenant en outre au moins un ingrédient supplémentaire parmi un antioxydant, un conservateur, un agent parfumant et un tensioactif.

10. Solution selon la revendication 9, où l'ingrédient supplémentaire est l'eucalyptol, l'acétate de tocophéryle, l'hydroxytoluène butylé, le BHA, le gallate de propyle, le palmitate d'ascorbyle, l'acide sorbique, l'huile d'eucalyptus, l'huile de cardamone, l'huile de lin, les huiles volatiles, ou le myristate d'isopropyle.
